# EUROPÄISCHE PATENTSCHRIFT

(11) **EP 2 389 859 B1**
(45) Veröffentlichungstag und Bekanntmachung des Hinweises auf die Patenterteilung: **12.08.2015**
(21) Anmeldenummer: 10164126.4
(22) Anmeldetag: 27.05.2010
(51) Int. Cl.: A61B 5/048, A61B 5/16, A61B 5/04

(54) **BCI-Vorrichtung zur Rehabilitation von Schlaganfallpatienten**
BCI device for use in stroke rehabilitation
Dispositif BCI pour la rééducation après un accident vasculaire cérébral

(43) Veröffentlichungstag der Anmeldung: 30.11.2011
(73) Patentinhaber: CorTec GmbH, 79110 Freiburg (DE)
(72) Erfinder: Rickert, Jörn, 79540, Lörrach (DE); Fischer, Jörg, 79276, Reute (DE)
(74) Vertreter: 2s | ip Schramm Schneider Patentanwälte Rechtsanwälte

(56) Entgegenhaltungen:
- US-A1- 2007 032 738
- US-B2- 7 120 486
- FEI MENG ET AL: "BCI-FES training system design and implementation for rehabilitation of stroke patients" 2008 INTERNATIONAL JOINT CONFERENCE ON NEURAL NETWORKS (IJCNN 2008), 1. Juni 2008 (2008-06-01), Seiten 4103-4106, XP031328124 ISBN: 978-1-4244-1820-6
- DALY J J, WOLPAW J R: "Brain-computer interfaces in neurological rehabilitation" LANCET NEUROLOGY, Bd. 7, Nr. 11, 1. November 2008 (2008-11-01), Seiten 1032-1043, XP025561313 ISSN: 1474-4422
- BALL TONIO ET AL: "Differential representation of arm movement direction in relation to cortical anatomy and function" JOURNAL OF NEURAL ENGINEERING, Bd. 6, Nr. 016006, Februar 2009 (2009-02), Seiten 1-16, XP002608910 ISSN: 1741-2560
- CHUANCHU WANG ET AL: "A feasibility study of non-invasive motor-imagery BCI-based robotic rehabilitation for stroke patients" PROCEEDINGS OF THE 4TH INTERNATIONAL IEEE EMBS CONFERENCE ON NEURAL ENGINEERING, 2009, 29. April 2009 (2009-04-29), Seiten 271-274, XP031478325 ISBN: 978-1-4244-2072-8

## Beschreibung

Die Erfindung betrifft eine BCI-Vorrichtung zur Unterstützung der Rehabilitation von Schlaganfallpatienten mit einer Sonde zur Ableitung eines neuronalen Aktivitätssignals, einer Auswertungseinheit zur Analyse des Aktivitätssignals sowie einem Effektor.

In den letzten Jahren hat das Interesse an BCI-Systemen (Brain-Computer-Interface) stark zugenommen. Ganz allgemein sind darunter Systeme zu verstehen, welche die direkte Ansteuerung von technischen Geräten durch Analyse der Aktivität des Gehirns ermöglichen, oder die umgekehrt eine Stimulation des Gehirns bewirken. Häufig wird der Begriff etwas eingeschränkter auf motorische Anwendungen in der Medizin bezogen, wo Patienten durch Prothesen oder eine Cursorsteuerung zumindest ein Teil der Selbständigkeit zurückgegeben werden soll, die durch eine gesundheitliche Beeinträchtigung eingebüßt wurde.

Es sind verschiedene Verfahren bekannt, die neuronale Aktivität eines Patienten zu messen. Dabei gibt es eine umgekehrte Wechselwirkung zwischen der Auflösung der erfassten Aktivitätsinformationen und dem Grad, bis zu dem invasiv in den Körper eingegriffen werden muss. An dem einen Ende der Skala stehen EEG (Elektroenzephalographie) oder MEG (Magnetoenzephalographie), die lediglich äußere Elektroden verwenden, dafür aber auch nur eine relativ grobe räumliche Auflösung ermöglichen. An dem anderen Ende der Skala können Aktionspotentiale einzelner Zellen (SUA, single unit activity) oder lokale Feldpotentiale (LFP, local field potentials) mit das Hirngewebe penetrierenden Elektroden aufgezeichnet werden. Das ermöglicht eine hohe räumliche Auflösung, verursacht aber praktisch eine ständig offene Wunde mit den entsprechenden Beschwerden und Infektionsgefahren. Außerdem ist es schwierig, langzeitstabile Ableitungen zu erreichen. Eine vermittelnde Lösung bieten ECoG-Elektroden, welche unter der Schädeldecke direkt auf die Hirnoberfläche aufgesetzt werden. Sie sind langzeitverträglich und liefern gegenüber EEG oder MEG deutlich besser räumlich aufgelöste neuronale Aktivitätsinformationen. Ein derartiges BCI wird in der US 7 120 486 B2 vorgestellt.

Die BCI-Vorrichtung in diesem Dokument umfasst einen Effektor in Form einer Anzeigeeinrichtung oder einer Prothese. Der Patient wird aufgefordert, sich eine bestimmte Bewegung vorzustellen bzw. zu versuchen, eine bestimmte Bewegung auszuführen. Das aufgezeichnete Aktivitätssignal wird mit einer Grundaktivität ohne die bestimmte Bewegung verglichen, und der Patient erhält mittels des Effektors eine Rückmeldung. Die Vorrichtung ist dazu ausgebildet, eine Mehrzahl von Frequenzkanälen zu bilden und das Aktivitätssignal anhand der Amplituden und/oder Phasen in den Frequenzkanälen zu bewerten, um das gewünschte Aktivitätsmuster zu identifizieren.

Die Arbeit von Tomo Ball und anderen, "Differential representation of arm movement direction in relation to cortical anatomy and function", J. Neural Eng. 2009:6 Art. Nr. 016006, untersucht, welche Frequenzbereiche einer ECoG-Sonde ein hohes bewegungsspezifisches Dekodiervermögen aufweisen.

Die weitaus meisten bisher diskutierten medizinischen Anwendungen richten sich an Patienten mit Lähmungen, bei denen eine organische Heilung nicht mehr möglich ist, obwohl der Kortex oder zumindest der Motorkortex als derjenige Bereich, der für die Kontrolle willkürlicher Bewegungen wesentlich ist, wenigstens noch teilweise intakt ist, die Nervenverbindungen zur Muskulatur aber unterbrochen sind. Das BCI-System ist demnach als reine Neuroprothese aufzufassen, welches eine natürliche Funktion des Körpers ersetzt, ohne sie zu heilen.

Solange noch eine Restbewegungsfähigkeit vorhanden ist, kann die Heilung durch gezielte Physiotherapie unterstützt werden. Ist aber ein Körperteil vollständig gelähmt, so bleibt der klassischen Medizin allenfalls noch der Versuch, das betroffene Körperteil passiv von außen zu bewegen. Das ist aber meist nicht ausreichend, um eine neuronale Reorganisation in den von dem Schlaganfall betroffenen Hirnarealen auszulösen, welche die Lähmung bessern könnte.

Ethan Buch und andere in "Think to Move: a Neuromagnetic Brain-Computer Interface (BCI) System for Chronic Stroke", Stroke 2008:39, Seiten 910-917, haben Schlaganfallpatienten beigebracht, eine gelähmte Hand mit Hilfe einer Orthese (orthopädische Prothese) zu öffnen oder zu schließen. Eine Orthese unterstützt ein Körperteil bei einer Bewegung, ersetzt es aber nicht. Zu deren Steuerung sollten die Patienten durch Entspannung eine Synchronisation oder Desynchronisation, also eine Stärkung oder Abschwächung, einer per MEG ausgewerteten neuronalen Aktivität erreichen, die dann in den entsprechenden Öffnungszustand der Orthese übersetzt wird. Zwar konnte die Ansteuerung erfolgreich gelernt werden, eine Verbesserung der Lähmung wurde aber nicht erzielt. Nachteilig hieran ist außerdem, dass die Patienten die Erzeugung künstlicher und nicht unbedingt für die Bewegung des gelähmten Körperteils spezifischer Hirnaktivität lernen mussten. Dazu wurde in diesem Fall die Mu-Aktivität im Band von 8-12 Hz ausgewertet, die sich wie die Beta-Aktivität im Band von 18-26 Hz verstärkt über den somatosensorischen und motorischen Kortices nachweisen lässt. Bewegung oder Bewegungsvorbereitung gehen oft mit einer Verringerung von Mu- und Beta-Rhythmen einher, während nach einer Bewegung oder bei Entspannung meist eine vorübergehende Rhythmusverstärkung erfolgt. Probanden können lernen, diese Aktivitäten gezielt zu beeinflussen, aber dies kostet sehr viel Konzentration und ist überdies sehr zeitaufwändig. Mu- und Betaaktivität zeigt zudem lediglich Bewegung als solche an, ist aber nicht geeignet, bestimmte gezielte Bewegungen oder Bewegungsabsichten zu dekodieren beziehungsweise vorherzusagen.

Janis Daly und Jonathan Wolpaw in "Brain-computer interfaces in neurological rehabilitation", Lancet Neurol 2008:7, Seiten 1032-1043, diskutieren den Gedanken, ohne Verwendung einer Orthese direkt bestimmte Aktivitätsmuster zu trainieren. Die Arbeit schweigt aber zu der Frage, welche Aktivitätsmuster dies sein und wie sie aus der abgeleiteten neuronalen Aktivität identifiziert werden könnten.

Eine weitere Idee aus Nick Ward, "The Neural Substrates of Motor Recovery After Focal Damage to the Central Nervous System", Arch. Phys. Med. Rehabil. 2006:87 Suppl. 2, Seiten S30-S35 besteht darin, den Motorkortex elektrisch zu stimulieren. Dies wird aber nicht mit der Identifizierung bestimmter neuronaler Aktivität des Patienten, sondern nur mit klassischer Physiotherapie in Beziehung gesetzt. Ein BCI-System zur Schlaganfallrehabilitation ist somit in keiner Weise angesprochen.

Die Arbeit von Fei Meng et al., "BCI-FES training system desing and implementation for rehabilitation of stroke patients", 2008 International Joint Conference on Neural Networks (IJCNN2008), 01.06.2008, S. 4103-4106 beschreibt ein Training für die Rehabilitation von chronischen Schlaganfallpatienten. Dazu wird in einem mehrkanaligen EEG die ereignisbezogene Synchronisation und Desynchronisation während der Vorstellung einer Handbewegung ausgewertet. Wird die vorgestellte Handbewegung erkannt, so wird eine funktionale elektrische Stimulation von für die Handbewegung zuständigen Muskeln ausgelöst.

Aus der US 2007/0032738 A1 ist ein adaptives Patiententraining für ein BCI bekannt, das auf unterschiedlichen Signalen wie EEG, ECoG oder LFP basieren kann.

Es ist bekannt, dass elektrische Stimulation ganz allgemein die Heilung von geschädigtem Hirngewebe fordern kann. Es gibt aber keinerlei Nachweise, dass eine Behandlung von Lähmungserscheinungen nach einem Schlaganfall allein durch elektrische Stimulation zu Erfolgen führt. Diese isolierte Stimulation wurde beispielsweise in einer Studie der Northstar Neuroscience Inc. versucht und führte nicht zu den gewünschten Ergebnissen (siehe z. B. unter http://www.reuters.com/article/idUSWNAS705820080122).

Daher ist Aufgabe der Erfindung, die Schlaganfallrehabilitation mit einem BCI zu unterstützen und damit neue Wege zur verbesserten Wiederherstellung zu eröffnen.

Diese Aufgabe wird durch eine BCI-Vorrichtung gemäß Anspruch 1 gelöst. Dabei geht die Erfindung von dem Grundgedanken aus, gezielt solche Aktivitätsmuster zu trainieren, die mit Bewegungen eines durch Schlaganfall gelähmten Körperteils in Verbindung stehen. Damit wird die Reorganisation des Gehirns gestützt, indem gezielt neuronale Aktivität geübt wird, die eine Wiederherstellung begünstigt. Dabei gibt die BCI-Vorrichtung rasch oder sogar in Echtzeit Rückmeldung, ob jeweils bei einem Trainingslauf eine derartige günstige neuronale Aktivität erkannt werden konnte oder nicht.

Dazu im Gegensatz wird bei vielen der herkömmlichen, als reine Neuroprothese eingesetzten BCI-Systemen nicht versucht, eine bestimmte neuronale Aktivität zu erzeugen, sondern lediglich beobachtet und versucht, daraus eine Bewegungsintention abzuleiten. Vorgegeben werden Bewegungen somit im Stand der Technik allenfalls während eines Trainings, damit das BCI-System Korrelationen zwischen neuronaler Aktivität und Bewegung einlernt. Im Verlauf eines solchen Trainings bekommt der Patient keine Rückmeldung; die BCI-Vorrichtung nimmt im Gegenteil die neuronalen Aktivitäten als Referenz hin, ohne sie zu bewerten.

Die Erfindung unterscheidet gezielt zwischen gewünschter neuronaler Aktivität, von der angenommen wird, dass sie die Rehabilitation unterstützt, und sonstiger neuronaler Aktivität. Soweit bei einigen bekannten EEG-basierten BCI-Systemen ein bestimmtes Aktivitätsmuster gelernt werden soll, etwa die Verstärkung von Mu-Rythmen, so ist dies künstlich und nur aufgrund des künstlichen Trainings mit der Bewegung verknüpft. Derartiges Training führt, wie die oben zitierte Arbeit von Ethan Buch und anderen belegt, zu keiner Reorganisation und Verbesserung der Lähmungserscheinungen. Erfindungsgemäß werden bestimmte Bewegungen vorgegeben und dekodiert, also beispielsweise Körperteil, Bewegungsrichtung, Bewegungsgeschwindigkeit und dergleichen. Das ist mit unspezifischen, nur mit Bewegung an sich korrelierten Signalen wie Mu- oder Beta-Rhythmen nicht möglich.

Der Begriff der Bewegung wird zunächst weit genug verwendet, um auch das Erzeugen von Sprache zu erfassen. Spezieller und besonders vorteilhaft durch die Erfindung unterstützt werden aber Bewegungen im engeren Sinne, also beispielsweise die Bewegung eines Armes, einer Hand, eines Beines oder eines anderen Körperteils.

Die Erfindung hat den Vorteil, dass Schlaganfallpatienten in wirksame Rehabilitationsmaßnahmen einbezogen werden können, die davon bislang ausgeschlossen waren. Dadurch ergibt sich eine verbesserte Wiederherstellung der Bewegungsfähigkeit mit dem zugehörigen Gewinn an Selbständigkeit und Lebensqualität.

Erfindungsgemäß ist der Effektor in Abhängigkeit von der Bewegung ansteuerbar und ist ausgestaltet, als Erfolgsrückmeldung das Gehirngewebe zu stimulieren. Die derart erzeugte Erfolgsrückmeldung ist vorteilhaft mit der bestimmten vorzustellenden oder auszuführenden Bewegung verknüpft, etwa indem gleichzeitig eine Orthese die reale Bewegung oder ein Avatar eine virtuelle Bewegung vollzieht. Es ist aber auch eine abstrakte Rückmeldung denkbar, wie eine Anzeige, welche den Grad der Übereinstimmung zwischen dem gemessenen Aktivitätssignal und dem gewünschten Aktivitätsmuster angibt.

Durch Ableitung mit zahlreichen Einzelelektroden, beispielsweise mindestens zwanzig, mindestens hundert oder sogar mindestens vierhundert Einzelelektroden, wird eine feine örtliche Auflösung des gemessenen Aktivitätssignals erzielt. Mit ECoG-Sonden lassen sich sogar Fingerbewegungen erkennen, besonders wenn sie aufgrund einer hohen Zahl von Einzelelektroden hochauflösend sind. Die erfindungsgemäße ECoG-Elektrode kann sowohl epidural als auch subdural sowie in einem Sulcus implantiert werden.

Das gewünschte Aktivitätsmuster wird anhand einer Amplituden- und/oder Phaseninformation in einer Mehrzahl von Frequenzkanälen, also mindestens zwei Frequenzkanälen identifiziert. Die Frequenzkanäle werden allgemein durch eine Fouriertransformation (beispielsweise FFT, Fast Fourier Transformation) bestimmt. Da nicht das ganze Spektrum aufgelöst werden muss, werden vereinfachte Schätzer eingesetzt. Um das Signal in den derart gebildeten Frequenzkanälen zu bewerten, wird beispielsweise über gleiche oder unterschiedliche Zeitfenster gemittelt.

Bevorzugt ist weiterhin ein Effektor in Form einer Orthese vorgesehen, welche für die Unterstützung oder Ausführung der bestimmten Bewegung an einem Körperteil ausgebildet ist. Durch die Verknüpfung zwischen der neuronalen Aktivität bei Bewegungsvorstellung oder dem Versuch der Bewegungsausführung und einer tatsächlichen, von außen herbeigeführten Bewegung wird das Gehirn besonders effektiv dazu angeregt, diese Verknüpfung auch durch Reorganisation nachzuvollziehen und so die Lähmungserscheinung abzubauen. Alternativ weist der Effektor eine Anzeigeeinrichtung auf, um die erfolgreiche Identifikation der bestimmten Bewegung darzustellen, insbesondere als Bildfolge der bestimmten Bewegung. Nicht immer steht eine geeignete Orthese zur Verfügung, so dass die Visualisierung der Bewegung der eigentlichen Bewegung noch am nächsten kommt.

Der Effektor, sei es eine Orthese oder eine Anzeigeeinrichtung oder ein anderes Mittel, welches dem Patienten Rückmeldung über den Grad des Erfolges der Ansteuerung gibt, reagiert in einer bevorzugten Ausführungsform zu Beginn der Rehabilitation, wenn das Gehirn noch stärker geschädigt ist, auf grob ähnliche Gehirnaktivität mit einer entsprechend grob ähnlichen Bewegung. Beispielsweise wird ein Korrelationskoeffizient bestimmt, welcher den Grad bewertet, in dem das gewünschte Aktivitätsmuster in dem Aktivitätssignal identifizierbar ist. Der Effektor wird mit einer entsprechenden Genauigkeit angesteuert. Zur besseren Motivation ist die von dem Effektor vollzogene Bewegung vorzugsweise immer ein bisschen genauer und besser als die neuronale Aktivität, es gibt also einen Vorlauf des Effektors. Da jeweils eine bestimmte Bewegung geübt wird, ist der BCI-Vorrichtung die zu vollziehende Bewegung in der systembedingt maximalen Genauigkeit bekannt. So wird beispielsweise zu Beginn der Rehabilitation nur eine rudimentäre Bewegung aufgrund einer Abweichung der neuronalen Aktivität von einem Grundrauschen ausgeführt, wobei die Stärke der Bewegung an eine Stärke der Abweichung angepasst ist. Später, wenn die Aktivität differenzierter wird und möglicherweise schon eine Teilbewegung regeneriert wurde, reagiert auch der Effektor auf spezifischere Aktivierungen mit spezifischeren Bewegungen, die mehr und mehr der vollständigen, richtigen Bewegung entsprechen.

Der Effektor weist bevorzugt einen Stimulator für Muskel gewebe auf, insbesondere zur Stimulation mit dem gewünschten Aktivitätsmuster. Stimulation des Muskelgewebes führt im besten Fall dazu, dass das gelähmte Körperteil mit künstlichen Signalen zu einer eigenständigen Bewegung veranlasst wird. Auch eine sensorische Rückmeldung aus dem gelähmten Körperteil kann für die Reorganisation hilfreich sein. Stimulation des Hirngewebes erfolgt vorzugsweise in der Umgebung der Läsion, um dort Aktivität anzuregen. Bei mangelndem Gefühl in dem zu rehabilitierenden Körperteil kann zusätzlich oder alternativ eine Stimulation durch eine weitere oder dieselbe ECoG-Elektrode erfolgen, die in den entsprechenden somatosensorischen Hirnarealen aufliegen. Dies unterstützt die Regeneration besonders dann, wenn diese somatosensorischen Hirnareale ebenfalls von dem Schlaganfall betroffen sind. Indem das gewünschte Aktivitätsmuster durch Stimulation präsentiert wird, lernt das Gehirn, dieses Aktivitätsmuster auch selbst wieder zu erzeugen. Alternativ ist aber auch inhibitorische Stimulation denkbar, beispielsweise eines kontralateralen Areals, um andere Hirnareale daran zu hindern, das gewünschte Aktivitätsmuster nachteilig zu beeinflussen.

In einer besonders bevorzugten Weiterbildung der Erfindung wird die Bewegung des Effektors mit einer Stimulation kombiniert. Dabei wird insbesondere die Stimulation an die Bewegung der Orthese gekoppelt. Wie der oben diskutierte Stand der Technik zeigt, lässt sich häufig weder mit Stimulation allein noch mit einer Orthesenbewegung allein eine hinreichende Rehabilitation erzielen. Durch die Kombination entsteht eine Assoziationskette zwischen Bewegungsvorstellung oder -steuerung, einem tatsächlichen, durch den Effektor bewirkten Bewegungserfolg und einer dazu passenden Stimulation des geschädigten Gehirngewebes, die insgesamt zu einer besonders erfolgreichen Reorganisation führt.

Dabei erzeugen bevorzugt verschiedene Bewegungen verschiedene bewegungsspezische Stimulationsmuster, welche die Orte am geschädigten Gewebe um die bewegungsauslösenden Elektroden stimulieren. Ein Beispiel für ein solches Stimulationsmuster ist das gewünschte Aktivitätsmuster oder ein davon abgeleitetes Aktivitätsmuster. Man versucht also, soweit dies mit einer oberflächlich aufliegenden oder in einen Sulcus eingeführten ECoG-Elektrode möglich ist, direkt die trainierten Aktivitätsmuster dem geschädigten Gehirnareal und dessen Umgebung aufzuprägen.

Die Auswertungseinheit ist bevorzugt dafür ausgebildet, nur für eine Anzahl ausgewählter Einzelelektroden Frequenzkanäle zu bilden, wobei für alle ausgewählten Einzelelektroden dieselben oder für unterschiedliche der ausgewählten Einzelelektroden unterschiedliche Frequenzkanäle gebildet werden. Dadurch werden nur Aktivitätssignale einbezogen, die einen Bezug zu der vorzustellenden oder auszuführenden Bewegung haben. Die Auswertung verursacht geringeren Aufwand und ist zugleich präziser, weil sich die bewegungsbezogene Aktivität weniger verdünnt. Im einfachsten Fall werden Elektroden ausgeschlossen, die gar kein Signal oder zu allen Zeiten ein gleichmäßiges Rauschsignal liefern. Spezifischere Kriterien finden in einem Training vor der eigentlichen Rehabilitationssitzung Verwendung. Für die derart ausgewählten Elektroden werden entweder die jeweils gleichen Frequenzkanäle gebildet, oder einigen ausgewählten Elektroden beziehungsweise jeder ausgewählten Elektrode werden individuell ein oder mehrere Frequenzkanäle zugewiesen. Insgesamt entsteht ein Parameterraum, der von der Summe der jeweiligen, ausgewählten Elektroden zugewiesenen Frequenzkanäle aufgespannt wird. Durch Auswertung von Amplitude und Phase oder Korrelationen zwischen diesen bisher genannten Parametern kann sich die Anzahl der Parameter noch einmal entsprechend vervielfachen. Indem das Vorhandensein des gewünschten Aktivitätsmusters auf den Patienten eingestellt wird, statt pauschal über alle Elektroden in demselben Frequenzbereich nach denselben Kriterien zu suchen, verringert sich der Auswertungsaufwand, während zugleich die Dekodierleistung und damit die Genauigkeit der Erkennung gewünschter Aktivität ansteigt. Dabei ist mit Dekodierleistung ein Maß dafür gemeint, wie selektiv und zuverlässig eine bewegungsselektive neuronale Aktivität erkannt wird.

Die Auswertungseinheit ist bevorzugt dafür ausgebildet, die Einzelelektroden und/oder die Frequenzkanäle zu den Einzelelektroden danach auszuwählen, dass sich das Aktivitätssignal bewegungsselektiv von einer Grundaktivität unterscheidet. Dabei werden Abweichungen nach oben oder unten anhand von Schwellwerten erkannt, die einer Signifikanzschwelle entsprechen. Es ist denkbar, auf eine Signifikanzschwelle zu verzichten, um die Anforderungen nicht zu überspannen und auch eine zufällige Abweichung durch positive Rückmeldung zu verstärken.

Die Auswertungseinheit ist bevorzugt dafür ausgebildet, einen niederfrequenten Frequenzkanal mit einer unteren Grenze von 0 Hz bis 2 Hz und einer oberen Grenze von 4 Hz bis 10 Hz und/oder einen hochfrequenten Frequenzkanal mit einer unteren Grenze von 50 Hz bis 70 Hz und einer oberen Grenze von 100 Hz bis 120 Hz oder mehr zu bilden. Es wurde nachgewiesen, dass bewegungsselektive Aktivität in diesen Frequenzkanälen eines ECoG-Signals besonders gut zu identifizieren ist. In dem ausgeschlossenen Band zwischen ca. 10 Hz und ca. 50 Hz, also auch in den herkömmlich verwendeten Mu- und Beta-Bändern, ist die Dekodierleistung dagegen geringer. Die ausgeschlossenen Bänder können unterstützend als reiner Bewegungsdetektor herangezogen werden, also um unabhängig von der bestimmten Bewegung überhaupt zu erkennen, dass eine Bewegung intendiert oder vollzogen wird. Eine für die bestimmte Bewegung spezifische Information ist den ausgeschlossenen Bändern aber nur in sehr begrenztem Maße oder überhaupt nicht zu entnehmen, so dass für die Decodierung der bestimmten Bewegung andere Frequenzen verwendet werden.

Man kann die untere Grenze des hochfrequenten Frequenzkanals weiter nach unten schieben als nur bis auf 50 Hz, aber dies führt zu keinen wesentlich verbesserten Ergebnissen. Die obere Grenze dagegen darf auch höher liegen als bei 120Hz.

Die Auswertungseinheit ist bevorzugt dafür ausgebildet, den hochfrequenten Frequenzkanal in einen unteren hochfrequenten Frequenzkanal mit einer unteren Grenze von 50 Hz bis 60 Hz und einer oberen Grenze von 90 Hz bis 100 Hz und einen oberen hochfrequenten Frequenzkanal mit einer unteren Grenze von mindestens 100 Hz und einer oberen Grenze von mindestens 200 Hz oberhalb der unteren Grenze zu bilden. Somit stehen insgesamt drei Frequenzkanäle und damit ein größerer Parameterraum zur Verfügung. Jedes Frequenzband mit Ausnahme des niederfrequenten Frequenzbandes sollte für eine hinreichende Dekodierleistung eine Breite von zumindest 10 Hz aufweisen.

Die Aufforderung zu der bestimmten Bewegung weist bevorzugt eine Richtungsangabe auf. In manchen Fällen besteht die Aufforderung lediglich in der Richtungsangabe. Diese lässt sich in Sektoren aufteilen, beispielsweise in die vier Hauptrichtungen eines orthogonalen Koordinatensystems oder die Sektoren einer Uhr, und aus dem neuronalen Aktivitätssignal relativ sicher erkennen. Die Aufforderung betrifft insbesondere eine Arm- oder Handbewegung. Die neuronale Aktivität während solcher Bewegungen ist verhältnismäßig gut verstanden, und die wiedergewonnene Einsatzfähigkeit der Hand hilft einem Patienten in besonderem Maße.

Die Auswertungseinheit ist bevorzugt für eine Auswertung in Echtzeit ausgebildet. Anders als bei klassischen Neuroprothesen ist eine Echtzeitauswertung nicht zwingend, so dass kürzere Verzögerungen beispielsweise für eine sicherere Erkennung des gewünschten Aktivitätsmusters in Kauf genommen werden können. Je schneller aber die Erfolgsrückmeldung kommt, desto befriedigender und effektiver ist die Rehabilitationsitzung für den Patienten.

Bevorzugt sind mehrere Ansätze für gewünschte Aktivitätsmuster vorgesehen. Sie können parallel oder alternierend eingesetzt werden, hängen aber auch von dem Grad der Beeinträchtigung des Kortex des Patienten ab. Ist der für die zu rehabilitierende Bewegung zuständige Kortex noch aktiv genug, um differenzierte Aktivitästmuster zu generieren, so können diese in einem anfänglichen Training eingelernt und dann gezielt geübt und verbessert werden. Ansonsten sind andere Referenzen heranzuziehen, von denen nachfolgend einige aufgeführt sind.

Dazu ist die Auswertungseinheit bevorzugt dafür ausgebildet, als gewünschtes Aktivitätsmuster jegliches Aktivitätssignal zu akzeptieren, welches sich von einer Grundaktivität ohne die bestimmte Bewegung unterscheidet. Prinzipiell sollte das gewünschte Aktivitätsmuster dasjenige sein, welches die Reorganisation bestmöglich unterstützt. Das ist beispielsweise eine Aktivität, die nahe an derjenigen ist, mit welcher der Patient das gelähmte Körperteil vor dem Schlaganfall bewegt hat. Dieses Aktivitätsmuster ist aber in aller Regel nicht bekannt. Außerdem kann das Gehirn es auch aufgrund der Läsion nicht mehr reproduzieren. Somit ist das gewünschte Aktivitätsmuster im Grunde das a priori unbekannte Aktivitätsmuster, welches sich nach erfolgreicher Rehabilitation einstellen wird. Als ersten Schritt in diese Richtung wird nun beobachtet, ob überhaupt irgendeine signifikante Abweichung in einer Grundaktivität und einer Aktivität während der Bewegungsvorstellung oder der versuchten Bewegung zu erkennen ist. Ein Spezialfall sind zuvor inaktive Einzelelektroden über der Läsion oder in der Nähe der Läsion. Jegliche hier auftretende Aktivität ist ein Zeichen für beginnende Reorganisation, die von der BCI-Vorrichtung durch die Erfolgsrückmeldung unterstützt wird. Dazu wird beispielsweise eine Standardbewegung beziehungsweise die aktuell aufgrund der Vorgabe zu übende Bewegung von dem Effektor vollzogen. In dieser Weise kann auch noch bei relativ starker Schädigung des beteiligten Gehirngewebes und wenig Wissen über die bewegungsspezifische Aktivität des Patienten vorgegangen werden.

Die Auswertungseinheit ist bevorzugt dafür ausgebildet, als gewünschtes Aktivitätsmuster ein Aktivitätssignal zu akzeptieren, das nicht mehr als ein Höchstmaß von einem Aktivitätssignal abweicht, welches in dem kontralateralen Gehirnbereich bei der bestimmten Bewegung des kontralateralen Körperteils trainiert wurde. Dies ist ein Ersatz für das nicht verfügbare Aktivitätssignal des von dem Schlaganfall betroffenen Hirngewebes, das von demselben Patienten bei einer analogen Bewegung stammt und deshalb einige signifikante Ähnlichkeiten aufweist. Beispielsweise sind die Frequenzbereiche, in denen sich bewegungsspezifische Aktivität zeigt, bei ein und derselben Person häufig in verschiedenen Regionen die gleichen. Um zumindest teilweise den invasiven Eingriff auf der an sich gesunden Gehirnhälfte zu vermeiden, kann anstelle eines Dauerimplantats nur kurzzeitig zur Aufzeichnung der neuronalen Aktivität implantiert werden. Weiterhin wird dort vorzugsweise nur epiduaral und nicht subdural implantiert oder sogar lediglich ein EEG-Signal verwendet. Anstelle eines Signals der patienteneigenen gesunden Hirnhälfte kann auch ein generisches Signal eines Gesunden als Referenz verwendet werden.

Eine vollständige Heilung des geschädigten Areals ist in den seltensten Fällen zu erwarten. Es ist deshalb unrealistisch anzunehmen, dass durch Rehabilitation zum gesunden Motorkortex identische neuronale Bewegungsaktivität antrainiert werden kann. Deshalb wird in einer besonders bevorzugten Ausführungsform ein Referenzsignal aus einem gesunden Hirnbereich des Patienten oder ein generisches, von Gesunden gewonnenes Referenzsignal nur anfänglich für die Rehabilitation verwendet. Sobald es gelungen ist, eine auch nur minimale Bewegungsfähigkeit des gelähmten Körpterteils zurückzugewinnen, werden in der weiteren Rehabilitation diejenigen Aktivitätsmuster geübt und stimuliert, welche diese Bewegungen erzeugen, um sie zu verstärken und zu differenzieren. Dadurch wird dem geschädigten Hirnareal kein vermeintlich richtiges Aktivitätsmuster aufgezwungen, welches aufgrund seiner Schädigung vielleicht gar nicht mehr erzeugbar ist, sondern es wird stattdessen eine natürliche Restrukturierung und Reorganisation des Gehirns zur Bahnung neuer Ansteuerungen des gelähmten Körperteils unterstützt.

Die Auswertungseinheit ist bevorzugt dafür ausgebildet, als gewünschtes Aktivitätsmuster ein Aktivitätssignal zu akzeptieren, welches bei Einzelelektroden in Nachbarschaft zu den ausgewählten Elektroden zu einem veränderten Aktivitätssignal führt. Die Elektroden wurden ausgewählt, weil sie bewegungsselektive Aktivität aufzeichnen. Wenn nun deren Nachbarn ebenfalls veränderte Aktivität zeigen, so ist dies ein Zeichen für eine beginnende Reorganisation in Abhängigkeit von der Bewegung. Die BCI-Vorrichtung unterstützt diese Reorganisation durch eine Erfolgsrückmeldung.

Die Auswertungseinheit ist bevorzugt dafür ausgebildet, als gewünschtes Aktivitätsmuster ein zuvor aufgezeichnetes Aktivitätssignal zu akzeptieren. Möglicherweise ist nämlich das frühere Aktivitätssignal des von der Läsion betroffenen Hirnareals aus einer Zeit vor der Läsion bekannt. Diese Aufzeichnung kann beispielsweise vor einem erneuten Schlaganfall bei mehrfachem Schlaganfall oder während einer Vorsorgeuntersuchung erfolgt sein. Alternativ handelt es sich um ein während eines Trainings ermitteltes Aktivitätssignal oder das Aktivitätssignal, welches während eines Teilerfolgs abgeleitet wurde. Solch ein Teilerfolg kann eine partielle Bewegung oder ein Zittern des gelähmten Körperteils oder einige Aktionspotentiale in dem gelähmten Körperteil sein. Damit werden Teilerfolge gesichert und verstärkt.

Die Sitzungsablauf-Steuerungseinheit ist bevorzugt für einen anfänglichen oder zyklischen Trainingsmodus ausgebildet, in dem die Frequenzkanäle, insbesondere auch die ausgewählten Elektroden und zugehörigen Frequenzkanäle, sowie Kriterien bewegungsselektiver Aktivitätssignale in den Frequenzkanälen insbesondere der ausgewählten Elektroden ermittelt werden. Damit wird die BCI-Vorrichtung an einen Patienten angepasst, um seine Rehabilitation individuell zu unterstützen.

Die BCI-Vorrichtung weist vorteilhafterweise eine Verstärker-/Stimulatoreinheit mit einer Befestigungseinrichtung am Körper des Patienten und mit einer Verbindung zu einem Computersystem mit der Auswertungseinheit auf, welches insbesondere mit dem als Anzeigeeinrichtung oder Orthese ausgebildeten Effektor verbunden ist, wobei die Sonde als Implantat zur dauerhaften Anordnung unterhalb der Schädeldecke ausgebildet ist und eine Drahtlosschnittstelle zur Verbindung mit einer Drahtlosschnittstelle der Verstärker-/Stimulatoreinheit aufweist. Durch die drahtlose Verbindung kann die Schädeldecke nach Implantieren der ECoG-Sonde vollständig verheilen. Die Verstärker-/Stimulatoreinheit ist bevorzugt extern und befindet sich in der Nähe des Patienten, beispielsweise in einer Umhängetasche oder einer Kopfbedeckung, damit der drahtlose Übertragungsweg kurz und störungsfrei bleibt. Sie kann relativ klein sein, da die erforderliche Rechenleistung in dem externen Computersystem bereitgestellt wird. Die Drahtlosschnittstelle versorgt das Implantat beispielsweise durch Induktion mit Strom beziehungsweise Spannung, so dass kein Austausch einer Energiequelle erforderlich ist.

Die Erfindung wird nachstehend auch hinsichtlich weiterer Merkmale und Vorteile anhand von Ausführungsbeispielen unter Bezugnahme auf die beigefügte Zeichnung näher erläutert. Die Figuren der Zeichnung zeigen in:
- Fig. 1: eine Übersichtsdarstellung eines erfindungsgemäßen BCI-Systems zur Schlaganfallrehabilitation;
- Fig. 2: eine Draufsicht auf eine ECoG-Elektrode zur Erläuterung von ausgewählten Einzelelektroden und zugehörigen Frequenzkanälen;
- Fig. 3: ein Diagramm, in dem die Dekodierleistung in Frequenzbändern zwischen einer auf der Y-Achse aufgetragenen unteren Grenze und einer auf der X-Achse aufgetragenen oberen Grenze dargestellt ist; und
- Fig. 4: ein Ablaufdiagramm eines Trainings und einer Rehabilitationssitzung.

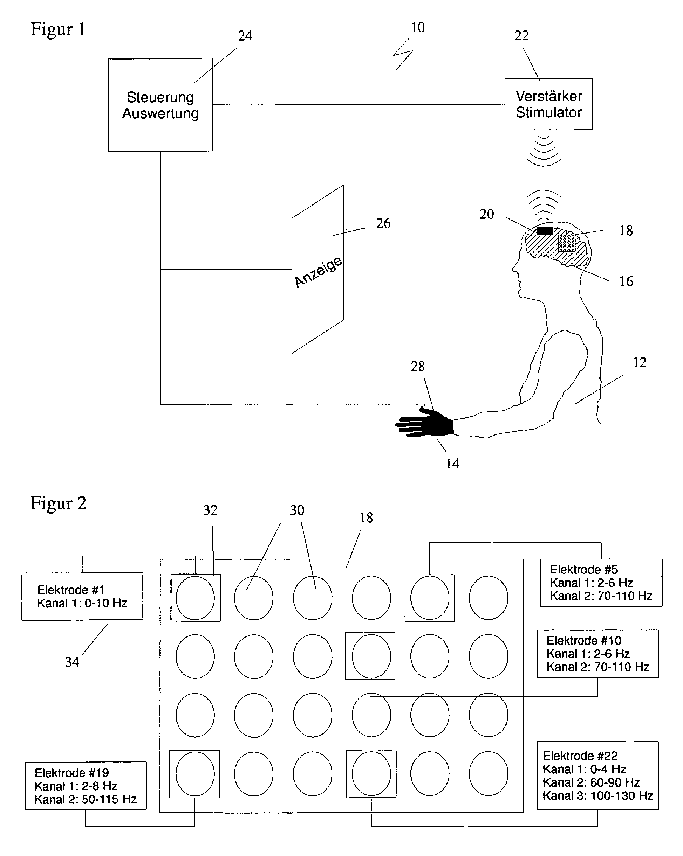

Figur 1 zeigt eine Übersichtsdarstellung eines erfindungsgemäßen BCI-Systems 10, welches die Rehabilitation eines von einem Schlaganfall betroffenen Patienten 12 unterstützt. Aufgrund des Schlaganfalls zeigt der Patient 12 Lähmungserscheinungen in einem Körperteil, welches im dargestellten Beispiel die linke Hand 14 ist. Ebenso kann aber der Arm, ein Bein oder ein beliebiges anderes Körperteil betroffen sein.

Auf dem Gehirn 16 des Patienten ist eine ECoG-Sonde 18 zur Ableitung der neuronalen Aktivität implantiert. Die ECoG-Sonde 18 wird innerhalb des Schädels auf die Hirnoberfläche aufgesetzt. Der Implantationsort wird durch allgemeine anatomische Kenntnisse oder durch präoperative Diagnose bestimmt. Vorzugsweise liegt die ECoG-Sonde 18 über dem Bereich des Motorkortex und des somatosensorischen Kortex, der für den gelähmten Körperteil 14 oder dessen Umgebung zuständig ist.

Die ECoG-Sonde 18 ist mit einer ebenfalls innerhalb des Schädels implantierten Drahtlosschnittstelle 20 verbunden. Grundsätzlich ist auch denkbar, eine Verbindungsleitung durch den Schädel zu führen, aber dies schränkt den Patienten 12 deutlich stärker ein. Die Drahtlosschnittstelle ist vorzugsweise bidirektional und kann einen Mikrocontroller zur Vorverarbeitung oder Schaltelemente zur Vorverstärkung der Signale von und zu der ECoG-Sonde 18 aufweisen.

In der Nähe des Patienten 12, beispielsweise in einer Kopfbedeckung, an dem Patientenbett oder in einer Umhängetasche, befindet sich eine Verstärker-/Stimulatoreinheit 22, die ein Gegenstück zu der Drahtlosschnittstelle 20 umfasst und somit Informationen und Befehle mit der implantierten ECoG-Sonde 18 oder dem Mikrocontroller der Drahtlosschnittstelle 20 austauschen kann. Über die Drahtlosschnittstelle 20 erfolgt auch die Versorgung des Implantats 18, 20 mit Strom beziehungsweise Spannung. Die Verstärker/Stimulatoreinheit 22 kann auch auf einem mobilen Gerät wie einem Handy oder einem PDA implementiert sein. Der Datenverkehr auf der Luftschnittstelle 20, 22 wird in einer Ausführungsform verschlüsselt beziehungsweise durch eine Authentifizierung gesichert, um versehentlichen oder absichtlichen Missbrauch auszuschließen.

Mit der Verstärker-/Stimulatoreinheit 22 ist eine Auswertungs- und Steuereinheit 24 verbunden. Dabei handelt es sich beispielsweise um einen Desktop-Computer, ein Notebook oder dergleichen. Prinzipiell ist denkbar, Verstärker-/Stimulatoreinheit 22 und Auswertungs- und Steuereinheit 24 in einem Gerät zu integrieren. Da aber für die Analyse der neuronalen Aktivität in der Auswertungs- und Steuereinheit 24 eine relativ große Rechenkapazität erforderlich ist, schränkt dies möglicherweise die Mobilität ein.

Die Auswertungs- und Steuereinheit 24 ihrerseits ist mit einer Anzeigeeinrichtung 26 und einer Orthese 28 verbunden, die als schwarze Unterlegung der Hand 14 dargestellt ist. Die Orthese 28 ist in der Lage, Bewegungen der gelähmten Hand 14 auszuführen oder zu unterstützen, beispielsweise eine Öffnungs- und Schließbewegung.

Figur 2 zeigt eine vergrößerte Draufsicht auf die ECoG-Sonde 18, die einen Träger aus biokompatiblem Material und eine Vielzahl von Einzelelektroden 30 aufweist. Das Layout der Einzelelektroden kann variieren, sogar durch individuelle Anpassung an den Patienten 12, und es ist eine unterschiedliche Anzahl von Einzelelektroden 30 von acht bis zweiunddreißig bis hin zu mehreren hundert Einzelelektroden 30 möglich. Meist sind Einzelelektroden 30 nur auf einer Seite des Trägers der ECoG-Sonde 18 vorgesehen. Es gibt aber auch die Möglichkeit, Einzelelektroden beidseitig auf dem Träger anzubringen und die ECoG-Sonde 18 in einem Sulcus (Furche) des Gehirns 16 zu implementieren. Dadurch werden zusätzliche Hirnbereiche zugänglich, wie in dem Patent DE 10 2006 008 501 B3 beschrieben.

Die Einzelelektroden 30 sind sowohl in der Lage, Aktivität des Gehirns 16 zu messen und ein entsprechendes Aktivitätssignal über die Drahtlosschnittstelle 20 auszugeben, als auch durch Ansteuerung mit einem Strom oder einer Spannung den darunterliegenden Bereich des Gehirns 16 zu stimulieren.

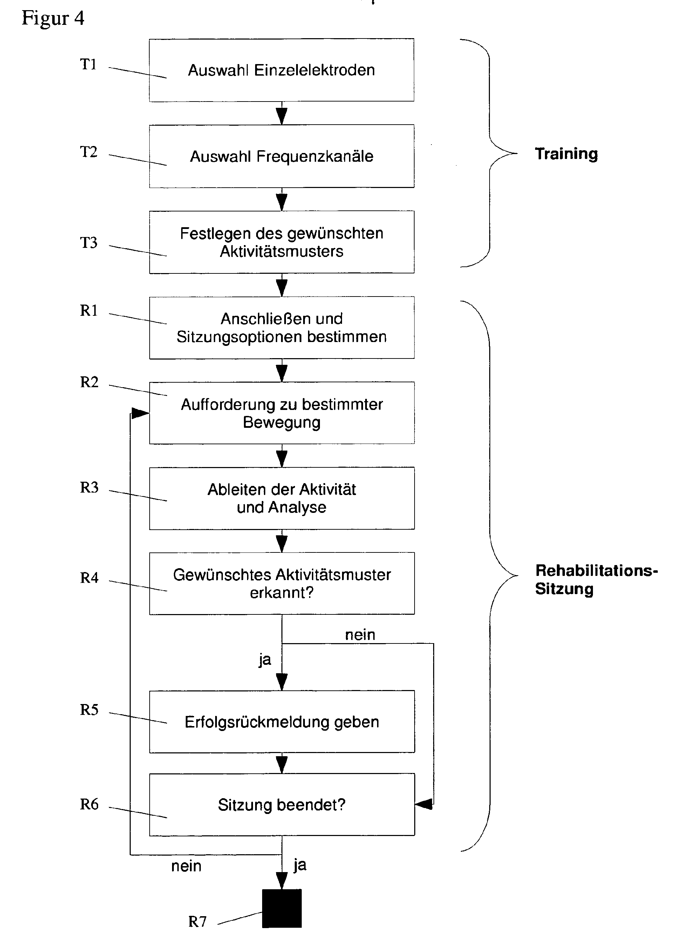

Nicht alle Einzelelektroden 30 tragen zu dem gemessenen Aktivitätssignal oder der Stimulation bei. Es werden in einem im Zusammenhang mit Figur 4 weiter unten beschriebenen Training oder durch den behandelnden Arzt Einzelelektroden 30 ausgewählt, die in besonderem Maße mit Bewegungen, sensorischen Reizen oder Bewegungsvorstellungen der gelähmten Hand stehen. Beispielhafte ausgewählte Einzelelektroden 32 sind in Figur 2 durch ein Rechteck markiert. Durch die Auswahl von Einzelelektroden 32 wird einerseits eine spezifischere Messung oder Stimulation erreicht. Andererseits entsteht auch weniger Datenverkehr an der Drahtlosschnittstelle 20, wenn die nicht ausgewählten Elektroden 30 bereits von dem Mikrocontroller des Implantats 18, 20 stumm geschaltet werden.

Es erfolgt aber nicht nur eine Auswahl von Einzelelektroden 32, sondern auch eine Zuweisung bestimmter Signaleigenschaften zu diesen Einzelelektroden 32. Dazu werden Frequenzkanäle gebildet und die Amplitude oder Phase in diesen Frequenzkanälen betrachtet. In Figur 2 sind rein beispielhaft einige Frequenzkanäle 34 gezeigt, welche den ausgewählten Elektroden 32 individuell zugewiesen werden. Alternativ ist denkbar, allen ausgewählten Elektroden 32 einheitliche Frequenzkanäle zuzuordnen.

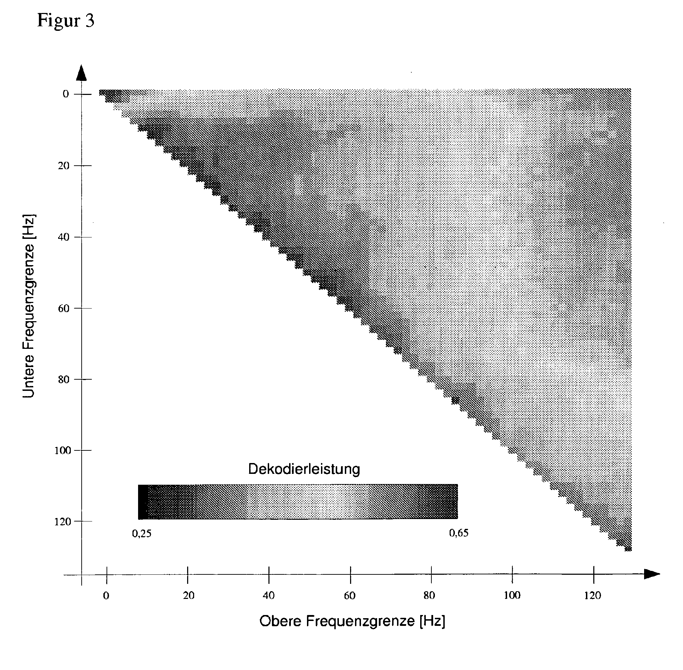

Figur 3 zeigt eine aus der oben zitierten Arbeit von Tonio Ball und anderen adaptierte Darstellung der Dekodierleistung in Abhängigkeit von der unteren und oberen Grenze eines Frequenzkanals. Daraus lassen sich Frequenzintervalle bestimmen, in denen die Aktivität besonders spezifisch mit Bewegungsvorstellungen oder Bewegungen der Hand 14 variiert. Beispielsweise ist Figur 3 zu entnehmen, dass Frequenzkanäle mit einer unteren Grenze bei ca. 10 Hz und einer oberen Grenze bei ca. 50 Hz nur eine sehr geringe Dekodierleistung haben. Ein solcher Frequenzkanal ist demnach keine günstige Wahl, um eine Bewegung oder Bewegungsabsicht zu erkennen. Dagegen gibt es andere Bereiche, beispielsweise einen niederfrequenten Kanal zwischen ca. 0 Hz und 8-10 Hz oder einen hochfrequenten Kanal zwischen ca. 60 Hz und 100 Hz oder einen Kanal oberhalb von 100 Hz, in denen die Dekodierleistung gemäß der Farbkodierung hoch ist, also eine Schwelle überschreitet. Durch günstige Wahl dieser Parameter wird die Bewertung der neuronalen Aktivität deutlich verbessert.

Die Verwendung und Funktionsweise des BCI-Systems 10 durch den Patienten 12 wird nun anhand des Ablaufschemas der Figur 4 näher erläutert. Dabei wird zwischen einer Trainingsphase und den eigentlichen Rehabilitationssitzungen unterschieden. Das Training wird anfänglich unter Begleitung des Arztes durchgeführt und dann nach gewissen Zeitintervallen oder auf Anforderung wiederholt. Allerdings muss nicht zwingend ein Training erfolgen, sondern es kann auch mit generischen Parametern gearbeitet werden.

In einem ersten Trainingsschritt T1 werden die ausgewählten Einzelelektroden 32 bestimmt. Zu den Auswahlkriterien zählt, dass eine Einzelelektrode 30 überhaupt ein Signal liefert, dass es sich von reinem Rauschen unterscheidet, dass es jeweils während einer Bewegung oder eines Bewegungsversuchs, einer Bewegungsvorstellung oder einer Bewegungsbeobachtung auftritt oder, im besten Fall, dass es spezifisch bestimmte Bewegungen oder Bewegungsvorstellungen anzeigt. Während des Trainings oder nach dessen Abschluss steht mehr Zeit für Auswertungen zur Verfügung als innerhalb einer Rehabilitationssitzung. Daher ist beispielsweise möglich, die Bewegungsvorhersage in der Steuer- und Auswertungseinheit 24 probehalber für verschiedene Gruppen von ausgewählten Einzelelektroden 32 zu simulieren und diejenige Auswahl weiterzuverwenden, welche die besten Ergebnisse liefert.

Anschließend werden in einem zweiten Trainingsschritt T2 den ausgewählten Elektroden 32 geeignete Frequenzkanäle zugewiesen. Diese Auswahl kann nach allgemeinen Kriterien erfolgen, wie sie in Figur 3 illustriert sind, aber auch individuell weiter an den Patienten 12 angepasst werden. Auch hier gilt nämlich wie bei der Auswahl der Einzelelektroden 32, dass der Steuer- und Auswertungseinheit 24 genug Rechenzeit zur Verfügung steht, durch aufwändige Verfahren oder Simulation einen Satz von Frequenzkanälen aufzufinden, welche individuell für den Patienten 12 die besten Ergebnisse erzeugt. Die Rechenleistung der Steuer- und Auswertungseinheit 24 ist durch zusätzliche Hardware oder eine Netzwerkverbindung zu weiteren Computern prinzipiell beliebig aufrüstbar.

In einem dritten Trainingsschritt T3 wird ein gewünschtes Aktivitätsmuster festgelegt, welches der Patient 12 durch die anschließenden Rehabilitationssitzungen lernen oder verstärken soll, um die Reorganisation zur Wiederherstellung der Bewegungsfähigkeit anzuregen oder zu beschleunigen. Dies ist also ein statisches oder dynamisches Muster in dem Parameterraum, der von Amplituden oder Phasen in den zugewiesenen Frequenzkanälen der ausgewählten Elektroden 32 aufgespannt wird, die beispielsweise jeweils über ein Zeitintervall gemittelt werden. Im einfachsten Fall wird das Muster nur durch Schwellbewertungen erkannt, also durch höhere oder geringere Werte als in einem Ruhezustand in den jeweiligen Parametern. Zu dem Parameterraum können auch Korrelationen dieser Größen über verschiedene ausgewählte Elektroden 32, Frequenzkanäle oder Zeiten gehören.

Es sind jeweils verschiedene Ansätze denkbar, welche Art von gewünschtem Aktivitätsmuster die Rehabilitation unterstützt, wobei diese Ansätze auch in Kombination oder abwechselnd einsetzbar sind.

In einem Ansatz wird vorab die Aktivität des korrespondierenden kontralateralen Gehirnbereichs bei entsprechender Bewegung bestimmt. Das wäre also in diesem Beispiel eine Bewegung der gesunden rechten Hand. Diese gibt Hinweise, welche Frequenzkanäle geeignet sind und wie deren Signalverlauf bei erfolgreicher Bewegung aussieht. Der Patient 12 soll anschließend lernen, ein Aktivitätsmuster mit dem von dem Schlaganfall betroffenen Hirnareal zu erzeugen, welches dem Aktivitätsmuster des gesunden gegenüberliegenden Hirnareals ähnelt. In diesem Fall kann alternativ zu den Schritten T1 und T2 mit einem zusätzlichen EEG-System die Aktivität des gesunden Areals gemessen und analysiert werden.

Ein sehr einfach zu handhabendes gewünschtes Aktivitätsmuster ist ein solches, welches überhaupt eine beliebige Aktivität oder Aktivitätsänderungen in der Nähe der Läsion erzeugt. Auch dieses Aktivitätsmuster wird in dem beschriebenen Parameterraum untersucht, ist also mit der Bewegung oder Bewegungsvorstellung der gelähmten Hand 14 korreliert. Da der Patient 12 die gelähmte Hand 14 im Alltag nicht mehr bewegen kann, fehlt es dem Gehirn 16 ohne Rehabilitationssitzungen an jeglicher Anregung für eine Reorganisation.

Ideal ist, wenn der Patient 12 schon erste Rehabilitationserfolge zeigt. Die dabei aufgezeichneten Änderungen der Aktivität oder deren Extrapolation werden dann durch weitere Rehabilitationssitzungen gezielt erhalten oder verstärkt.

Sobald ein durch die Rehabilitationssitzung zu erlernendes oder zu vertiefendes gewünschtes Aktivitätsmuster festliegt, ist das Training abgeschlossen. Die in den Schritten T1 bis T3 getroffene Wahl kann aber auch zu einem späteren Zeitpunkt verändert werden.

Während einer direkt oder mit zeitlichem Abstand auf das Training folgenden Rehabilitationssitzung wird in einem ersten Schritt R1 die Steuer- und Auswertungseinheit 24 mit der Verstärker/Stimulatoreinheit 22 verbunden, und beide werden aktiviert. Über die Drahtlosschnittstelle 20 wird auch das Implantat 18, 20 in Betriebsbereitschaft versetzt. Optional werden Sitzungsparameter eingestellt, etwa ein bestimmter Patient 12 ausgewählt sowie die Anzahl der Wiederholungen oder eine Sitzungsdauer bestimmt.

In einem zweiten Schritt R2 fordert die Steuer- und Auswertungseinheit 24 den Patienten 12 über die Anzeigeeinrichtung 26 dazu auf, sich eine bestimmte Bewegung der gelähmten Hand 14 vorzustellen oder zu versuchen, diese Bewegung mit der gelähmten Hand 14 zu vollziehen. Dabei handelt es sich beispielsweise um eine Öffnungs- oder Schließbewegung. Der Patient 12 soll also keinerlei künstliche Hirnaktivität erzeugen, sondern lediglich die Aktivität, die mit einer ganz spezifischen natürlichen Bewegung verbunden wäre. Ziel der Rehabilitation ist, eine Reorganisation zu erreichen, demnach die noch vorhandenen neuronalen Pfade auszunutzen, zu ergänzen oder zu reparieren.

Beginnend mit der Aufforderung leitet die ECoG-Sonde 18 in einem dritten Schritt R3 die neuronale Aktivität ab und sendet entsprechende Signale der ausgewählten Elektroden 32 über die Drahtlosschnittstelle 20 zu der Verstärker-/Stimulatoreinheit 22. Von dort werden die verstärkten Signale an die Steuer- und Auswertungseinheit 24 übergeben und dort analysiert. Dabei werden in Echtzeit oder nahezu in Echtzeit die notwendigen Transformationen, Mittelungen und Korrelationen gebildet, um die während des Trainings gewählten Parameter auswerten zu können.

Die Steuer- und Auswertungseinheit 24 entscheidet in einem vierten Schritt R4 anhand dieser analysierten Aktivitätsdaten, ob die neuronale Aktivität des Patienten 12 dem gewünschten Aktivitätsmuster entspricht. Wenn das nicht der Fall ist, war die aktuelle Übung innerhalb der Rehabilitationssitzung nicht erfolgreich. Darüber kann dem Patienten 12 optional eine kurze Rückmeldung über die Anzeigeeinrichtung 26 gegeben werden.

Wird das gewünschte Aktivitätsmuster erkannt, so erfolgt in einem fünften Schritt R5 eine Erfolgsrückmeldung. Erfindungsgemäß erfolgt eine Stimulation des Gehirns 18 als Erfolgsrückmeldung. Dazu sendet die Verstärker-/Stimulatoreinheit 22 einen entsprechenden Befehl an die Drahtlosschnittstelle 20, so dass Einzelelektroden 32 der ECoG-Sonde 18 das darunterliegende Gehirngewebe stimulieren. Wie schon bei dem gewünschten Aktivitätsmuster sind auch für die Stimulation mehrere Ansätze möglich. So kann das Gewebe im Bereich der Läsion oder dessen Umgebung stimuliert werden. Alternativ wird ein zugehöriger somatosensorischer Bereich stimuliert, oder die entsprechenden Areale der gesunden rechten Hand in der kontralateralen Gehirnhälfte. Speziell wird versucht, das gewünschte Aktivitätsmuster durch Stimulation nachzubilden und so noch weiter zu verstärken. Da die ECoG-Elektrode an der Oberfläche aufliegt, ist dies nur in Grenzen möglich. Tiefere Gewebeschichten werden durch eine ECoG-Elektrode erreicht, die in einem Sulcus sitzt. Zusätzlich sind verschiedene Varianten denkbar, die gleichzeitig oder alternativ zueinander zum Einsatz kommen können. Eine sehr schlichte Erfolgsrückmeldung besteht in einer Meldung auf der Anzeigeeinrichtung 26. Etwas eindrücklicher ist, wenn dies in Form einer grafischen Darstellung der gewünschten Bewegung eines Avatars geschieht.

Eine natürlichere Rückmeldung ist der Vollzug der in der Aufforderung bezeichneten Bewegung mittels der Orthese 28. Bestand beispielsweise die Aufforderung darin, die Hand 14 zu öffnen, so streckt die Orthese 28 die Finger in entsprechender Weise aus. Durch die Verknüpfung der Bewegungsvorstellung oder dem neuronalen Bewegungsbefehl mit einem Aktivitätsmuster, das mit dieser Bewegung in einer Beziehung steht, und dem anschließenden Vollzug entsteht genau die Kette von Ereignissen, die zur Linderung der Lähmung erzielt werden soll.

Mit der Erfolgsrückmeldung ist die aktuelle Übung beendet. In einem sechsten Schritt wird geprüft, ob damit das Ende der Rehabilitationssitzung erreicht ist. Dementsprechend wird die Rehabilitationssitzung bei dem zweiten Schritt R2 fortgesetzt oder in einem siebten Schritt R7 beendet.

In der beschriebenen Weise können eine oder mehrere spezifische Bewegungen gezielt trainiert und zurückgewonnen werden. Für diese Bewegungen werden mit einer Rückmeldung über Orthese 28 und bevorzugt zugleich auch einer Stimulation mittels des Stimulators 22 Verbindungen des Kortex zu der Muskulatur gebahnt oder verstärkt.

Sobald zumindest eine Bewegung in Ansätzen gelernt wurde, kann anstelle einer gezielten Rehabilitationssitzung mit vorgegebenen Bewegungen in einen freien Modus des BCI-Systems 10 übergegangen werden. Dabei entscheidet der Patient selbst, welche Bewegung er ausführt. Das BCI-System 10 erkennt diese Bewegung, um sie beispielsweise durch die Orthese 28 zu unterstützen oder durch Stimulation mit Hilfe des Stimulators 22 zu verstärken. Wenn der Patient von Anfang an noch in der Lage ist, bewegungsspezifische neuronale Aktivität zu erzeugen, ist auch möglich, die Rehabilitation unmittelbar in diesem freien Modus zu beginnen. Wenn dabei die Orthese 28 vorläuft, also die intendierte Bewegung mit größerer Präzision unterstützt, als der Patient allein sie erzeugen könnte, gewinnt der Patient sofort zusätzliche Bewegungsmöglichkeiten und empfindet die Rehabilitation im Idealfall von Anfang an als Unterstützung seines Alltags. Damit steigt nicht nur die Lebensqualität, sondern auch die Rehabilitation ist erfolgreicher, weil der Patient das System von sich aus in einem höheren Maße nutzt.

## Patentansprüche

1. BCI-Vorrichtung (10) zur Unterstützung der Rehabilitation von Schlaganfallpatienten (12) mit Lähmungserscheinungen, die eine Sonde (18) zur Ableitung eines neuronalen Aktivitätssignals, eine Auswertungseinheit (24) zur Analyse des Aktivitätssignals sowie einen Effektor (18, 22) aufweist, welcher von der Auswertungseinheit (24) in Abhängigkeit von einer erkannten bestimmten Bewegung ansteuerbar ist, wobei eine Sitzungsablauf-Steuerungseinheit (24) dafür ausgebildet ist, eine Aufforderung an den Patienten (12) auszugeben, ein gewünschtes Aktivitätsmuster zu erzeugen, dabei mit Hilfe der Sonde (18) das Aktivitätssignal aufzuzeichnen und immer dann, wenn von der Auswertungseinheit (24) das gewünschte Aktivitätsmuster identifizierbar ist, dem Patienten (12) darüber mittels des Effektors (18, 22) Rückmeldung zu geben,
wobei die Aufforderung eine Anweisung umfasst, sich die bestimmte Bewegung vorzustellen oder zu versuchen, die bestimmte Bewegung auszuführen, wobei die Sonde (18) eine ECoG-Elektrode mit mindestens acht Einzelelektroden (30) aufweist und
wobei die Auswertungseinheit (24) dafür ausgebildet ist, für die Identifikation des gewünschten Aktivitätsmusters eine Mehrzahl von Frequenzkanälen zu bilden und das Aktivitätssignal anhand der Amplituden und/oder Phasen in den Frequenzkanälen zu bewerten und eine Erfolgsrückmeldung auszugeben, wenn das gewünschte Aktivitätsmuster identifiziert wird,
wobei der Effektor (24) einen Stimulator (22, 18) für Gehirngewebe aufweist und ausgestaltet ist, als Erfolgsrückmeldung das Gehirngewebe zu stimulieren .

2. BCI- Vorrichtung (10) nach Anspruch 1, welche zusätzlich einen Effektor in Form einer Orthese (28) aufweist, welche für die Unterstützung oder Ausführung der bestimmten Bewegung an einem Körperteil (14) ausgebildet ist, oder eine Anzeigeeinrichtung (26) aufweist, um die erfolgreiche Identifikation der bestimmten Bewegung darzustellen, insbesondere als Bildfolge der bestimmten Bewegung.

3. BCI- Vorrichtung (10) nach Anspruch 1 oder 2,
wobei der Stimulator (22, 18) zur Stimulation mit dem gewünschten Aktivitätsmuster ausgestaltet ist.

4. BCI- Vorrichtung (10) nach einem der vorhergehenden Ansprüche,
wobei die Auswertungseinheit (24) dafür ausgebildet ist, nur für eine Anzahl ausgewählter Einzelelektroden (32) Frequenzkanäle zu bilden, wobei für alle ausgewählten Einzelelektroden (32) dieselben oder für unterschiedliche der ausgewählten (32) Einzelelektroden unterschiedliche Frequenzkanäle gebildet werden.

5. BCI- Vorrichtung (10) nach Anspruch 4,
wobei die Auswertungseinheit (24) dafür ausgebildet ist, die Einzelelektroden (30) und/oder die Frequenzkanäle zu den Einzelelektroden (30) danach auszuwählen, dass sich das Aktivitätssignal bewegungsselektiv von einer Grundaktivität unterscheidet.

6. BCI- Vorrichtung (10) nach einem der vorhergehenden Ansprüche,
wobei die Auswertungseinheit (24) dafür ausgebildet ist, einen niederfrequenten Frequenzkanal mit einer unteren Grenze von 0 Hz bis 2 Hz und einer oberen Grenze von 4 Hz bis 10 Hz und/oder einen hochfrequenten Frequenzkanal mit einer unteren Grenze von 50 Hz bis 70 Hz und einer oberen Grenze von 100 Hz bis 120 Hz oder mehr zu bilden.

7. BCI- Vorrichtung (10) nach Anspruch 6,
wobei die Auswertungseinheit (24) dafür ausgebildet ist, den hochfrequenten Frequenzkanal in einen unteren hochfrequenten Frequenzkanal mit einer unteren Grenze von 50 Hz bis 60 Hz und einer oberen Grenze von 90 Hz bis 100 Hz und einen oberen hochfrequenten Frequenzkanal mit einer unteren Grenze von mindestens 100 Hz und einer oberen Grenze von mindestens 20 Hz oberhalb der unteren Grenze zu bilden.

8. BCI- Vorrichtung (10) nach einem der vorhergehenden Ansprüche,
wobei die Aufforderung zu der bestimmten Bewegung eine Richtungsangabe aufweist und insbesondere eine Arm- oder Handbewegung betrifft.

9. BCI- Vorrichtung (10) nach einem der vorhergehenden Ansprüche,
wobei die Auswertungseinheit (24) für eine Auswertung in Echtzeit ausgebildet ist.

10. BCI- Vorrichtung (10) nach einem der vorhergehenden Ansprüche,
wobei die Auswertungseinheit (24) dafür ausgebildet ist, als gewünschtes Aktivitätsmuster jegliches Aktivitätssignal zu akzeptieren, welches sich von einer Grundaktivität ohne die bestimmte Bewegung unterscheidet.

11. BCI- Vorrichtung (10) nach einem der vorhergehenden Ansprüche,
wobei die Auswertungseinheit (24) dafür ausgebildet ist, als gewünschtes Aktivitätsmuster ein Aktivitätssignal zu akzeptieren, das nicht mehr als ein Höchstmaß von einem Aktivitätssignal abweicht, welches in dem kontralateralen Gehirnbereich bei der bestimmten Bewegung des kontralateralen Körperteils trainiert wurde.

12. BCI- Vorrichtung (10) nach einem der vorhergehenden Ansprüche,
wobei die Auswertungseinheit (24) dafür ausgebildet ist, als gewünschtes Aktivitätsmuster ein Aktivitätssignal zu akzeptieren, welches bei Einzelelektroden (30) in Nachbarschaft zu den ausgewählten Elektroden (32) zu einem veränderten Aktivitätssignal führt.

13. BCI- Vorrichtung (10) nach einem der vorhergehenden Ansprüche,
wobei die Auswertungseinheit (24) dafür ausgebildet ist, als gewünschtes Aktivitätsmuster ein zuvor aufgezeichnetes Aktivitätssignal zu akzeptieren.

14. BCI- Vorrichtung (10) nach einem der vorhergehenden Ansprüche,
wobei die Sitzungsablauf-Steuerungseinheit (24) für einen anfänglichen oder zyklischen Trainingsmodus ausgebildet ist, in dem die Frequenzkanäle, insbesondere auch die ausgewählten Elektroden (32) und zugehörige Frequenzkanäle, sowie Kriterien bewegungsselektiver Aktivitätssignale in den Frequenzkanälen insbesondere der ausgewählten Elektroden (32) ermittelt werden.

15. BCI- Vorrichtung (10) nach Anspruch 2, die eine Verstärker-/Stimulatoreinheit (22) mit einer Befestigungseinrichtung am Körper des Patienten (12) und mit einer Verbindung zu einem Computersystem (24) mit der Auswertungseinheit aufweist, welches insbesondere mit der Anzeigeeinrichtung (26) oder Orthese (28) verbunden ist, wobei die Sonde (18) als Implantat zur dauerhaften Anordnung unterhalb der Schädeldecke ausgebildet ist und eine Drahtlosschnittstelle (20) zur Verbindung mit einer Drahtlosschnittstelle der Verstärker-/Stimulatoreinheit (22) aufweist.

## Claims

1. A BCI device (10) for supporting the rehabilitation of stroke patients (12) showing paralysis symptoms, said device having a probe (18) for deriving a neuronal activity signal, an evaluation unit (24) for analysing the activity signal, and an effector (18, 22) which may be activated by the evaluation unit (24) upon detection of a specific movement, wherein a session flow control unit (24) is adapted for issuing a request to the patient (12) to produce a desired activity pattern, for recording the corresponding activity signal using the probe (18), and, whenever the desired activity pattern is identifiable by the evaluation unit (24), for providing feedback to the patient (12) by means of the effector (18, 22),
wherein the request comprises an instruction to imagine the specific movement or to try to carry out the specific movement, the probe (18) having an ECoG electrode with at least eight individual electrodes (30), and
wherein the evaluation unit (24) is adapted for forming a plurality of frequency channels for identifying the desired activity pattern and for evaluating the activity signal on the basis of the amplitudes and/or phases in the frequency channels, and for providing positive feedback if the desired activity pattern is being ictentified,
wherein the effector (24) has a brain tissue stimulator (22, 18) and is configured for providing positive feedback by stimulating said brain tissue.

2. The BCI device (10) as claimed in claim 1, further including an effector in the form of an orthosis (28) which is adapted for supporting, or carrying out, the specific movement of a body part (14), or including a display device (26) in order to represent the successful identification of the specific movement, in particular as an image sequence of the specific movement.

3. The BCI device (10) as claimed in claim 1 or 2, wherein the stimulator (22, 18) is configured for stimulation purposes with the desired activity pattern.

4. The BCI device (10) as claimed in any of the preceding claims,
wherein the evaluation unit (24) is adapted for forming frequency channels only for a number of selected, individual electrodes (32), with the same frequency channels being formed for all of the selected, individual electrodes (32) or with different frequency channels being formed for different ones of the selected, individual electrodes (32).

5. The BCI device (10) as claimed in claim 4,
wherein the evaluation unit (24) is adapted for selecting the individual electrodes (30) and/or the frequency channels corresponding to the individual electrodes (30) in such a manner that the selectively movement-specific activity signal differs from that of a base activity.

6. The BCI device (10) as claimed in any of the preceding claims,
wherein the evaluation unit (24) is adapted for forming a low-frequency channel having a lower limit of between 0 Hz and 2 Hz and an upper limit of between 4 Hz and 10 Hz and/or a high-frequency channel having a lower limit of between 50 Hz and 70 Hz, and an upper limit of between 100 Hz and 120 Hz or more.

7. The BCI device (10) as claimed in claim 6,
wherein the evaluation unit (24) is adapted for forming the high-frequency channel into a lower high-frequency channel having a lower limit of between 50 Hz and 60 Hz and an upper limit of between 90 Hz and 100 Hz and into an upper high-frequency channel having a lower limit of at least 100 Hz and an upper limit of at least 20 Hz above the lower limit.

8. The BCI device (10) as claimed in any of the preceding claims,
wherein the request concerning the specific movement includes a directional indication and particularly relates to a movement of the arm or the hand.

9. The BCI device (10) as claimed in any of the preceding claims,
wherein the evaluation unit (24) is adapted for performing a real-time evaluation.

10. The BCI device (10) as claimed in any of the preceding claims,
wherein the evaluation unit (24) is adapted for accepting as a desired activity pattern any activity signal that is different from that of a base activity without the specific movement.

11. The BCI device (10) as claimed in any of the preceding claims,
wherein the evaluation unit (24) is adapted for accepting as a desired activity pattern an activity signal which does not deviate by more than a given maximum amount from an activity signal used for training purposes in the contralateral brain area upon performing the specific movement of the contralateral body part.

12. The BCI device (10) as claimed in any of the preceding claims,
wherein the evaluation unit (24) is adapted for accepting as a desired activity pattern an activity signal that causes a changed activity signal in individual electrodes (30) located adjacent to the selected electrodes (32).

13. The BCI device (10) as claimed in any of the preceding claims,
wherein the evaluation unit (24) is adapted for accepting as a desired activity pattern a previously recorded activity signal.

14. The BCI device (10) as claimed in any of the preceding claims,
wherein the session flow control unit (24) is adapted for an initial or cyclic training mode in which the frequency channels, including in particular the selected electrodes (32) and associated frequency channels, as well as criteria for selectively movement-specific activity signals in the frequency channels, in particular of the selected electrodes (32) are determined.

15. The BCI device (10) as claimed in claim 2, having an amplifier/stimulator unit (22) provided with a fastening device for fastening onto the body of the patient (12) and with a connection to a computer system (24) running the evaluation unit and connected, in particular, to a display device (26) or orthosis (28), the probe (18) being adapted as an implant for permanent arrangement below the skull cap and provided with a wireless interface (20) designed for connecting with a wireless interface of the amplifier/stimulator unit (22).

## Revendications

1. Dispositif BCI (10) destiné à assister la rééducation de patients (12) victimes d'un accident vasculaire cérébral avec signes de paralysie, lequel dispositif présente une sonde (18) destinée à dévier un signal d'activité neuronale, une unité d'évaluation (24) destinée à analyser ledit signal d'activité ainsi qu'un effecteur (18, 22) qui peut être activé par l'unité d'évaluation (24) en fonction d'un mouvement spécifique détecté, l'unité de commande du déroulement des séances (24) étant conçue pour émettre à l'irïtention du patient une demande l'invitant à réaliser un schéma d'activité souhaité tout en enregistrant le signal d'activité à l'aide de la sonde (18) et, chaque fois que le schéma d'activité souhaité a pu être identifié par l'unité d'évaluation (24), d'en informer en retour le patient (12) au moyen de l'effecteur (18, 22),
dans lequel la demande comprend une instruction invitant le patient à se représenter le mouvement spécifique ou à essayer d'effectuer le mouvement spécifique, la sonde (18) présentant une électrode ECoG pourvue d'au moins huit électrodes individuelles (30) et
dans lequel l'unité d'évaluation (24) est conçue pour former une pluralité de canaux de fréquence en vue de l'identification du schéma d'activité souhaité et évaluer le signal d'activité grâce aux amplitudes et/ou aux phases dans les canaux de fréquence et pour émettre en retour un message de réussite lorsque le schéma d'activité souhaité est identifié,
dans lequel l'effecteur (24) présente un stimulateur (22, 18) pour tissu cervical et est conçu pour stimuler en retour, en tant que message de réussite, le tissu cervical.

2. Dispositif BCI (10) selon la revendication 1 lequel présente en outre un effecteur réalisé sous la forme d'une orthèse (28) qui est conçue pour assister ou effectuer le mouvement spécifique au niveau d'une partie de corps donnée (14), ou lequel présente un système d'affichage (26) afin de représenter l'identification réussie du mouvement spécifique, en particulier en tant que séquence d'images du mouvement spécifique.

3. Dispositif BCI (10) selon la revendication 1 ou 2 dans lequel le stimulateur (22, 18) est conçu, pour la stimulation, avec le schéma d'activité souhaité.

4. Dispositif BCI (10) selon l'une quelconque des revendications précédentes,
dans lequel l'unité d'évaluation (24) est conçue pour former des canaux de fréquence uniquement pour un nombre donné d'électrodes individuelles sélectionnées (32), les mêmes canaux de fréquence étant formés pour toutes les électrodes individuelles sélectionnées (32) ou des canaux de fréquence différents étant formés pour différentes électrodes individuelles sélectionnées (32).

5. Dispositif BCI (10) selon la revendication 4,
dans lequel l'unité d'évaluation (24) est conçue pour sélectionner les électrodes individuelles (30) et/ou les canaux de fréquence correspondant aux électrodes individuelles (30) en fonction du fait que le signal d'activité diffère de l'activité de base, et ce de manière sélectivement spécifique au mouvement.

6. Dispositif BCI (10) selon l'une quelconque des revendications précédentes,
dans lequel l'unité d'évaluation (24) est conçue pour former un canal de fréquence à basse fréquence avec une limite inférieure comprise entre 0 Hz et 2 Hz et une limite supérieure comprise entre 4 Hz et 10 Hz et/ou un canal de fréquence à haute fréquence avec une limite inférieure comprise entre 50 Hz et 70 Hz et une limite supérieure comprise entre 100 Hz et 120 Hz ou plus.

7. Dispositif BCI (10) selon la revendication 6,
dans lequel l'unité d'évaluation (24) est conçue pour former dans le canal de fréquence à haute fréquence un canal à haute fréquence inférieur avec une limite inférieure comprise entre 50 Hz et 60 Hz et une limite supérieure comprise entre 90 Hz et 100 Hz et un canal à haute fréquence supérieur avec une limite inférieure d'au moins 100 Hz et une limite supérieure située à au moins 20 Hz au-dessus de la limite inférieure.

8. Dispositif BCI (10) selon l'une quelconque des revendications précédentes,
dans lequel la demande relative au mouvement spécifique présente une indication de direction et concerne en particulier un mouvement de bras ou de main.

9. Dispositif BCI (10) selon l'une quelconque des revendications précédentes,
dans lequel l'unité d'évaluation (24) est conçue pour une évaluation en temps réel.

10. Dispositif BCI (10) selon l'une quelconque des revendications précédentes,
dans lequel l'unité d'évaluation (24) est conçue pour accepter comme schéma d'activité souhaité tout signal d'activité qui diffère de celui d'une activité de base sans le mouvement spécifique.

11. Dispositif BCI (10) selon l'une quelconque des revendications précédentes,
dans lequel l'unité d'évaluation (24) est conçue pour accepter comme schéma d'activité souhaité un signal d'activité qui ne diffère pas plus que d'une valeur maximum donnée d'un signal d'activité apparaissant dans la zone cervicale contralatérale lors de l'apprentissage du mouvement spécifique de la partie de corps contralatérale.

12. Dispositif BCI (10) selon l'une quelconque des revendications précédentes,
dans lequel l'unité d'évaluation (24) est conçue pour accepter comme schéma d'activité souhaité un signal d'activité qui provoque une modification du signal d'activité au niveau d'électrodes individuelles (30) situées au voisinage des électrodes sélectionnées (32).

13. Dispositif BCI (10) selon l'une quelconque des revendications précédentes,
dans lequel l'unité d'évaluation (24) est conçue pour accepter comme schéma d'activité souhaité un signal d'activité préalablement enregistré.

14. Dispositif BCI (10) selon l'une quelconque des revendications précédentes,
dans lequel l'unité de commande du déroulement des séances (24) est conçue pour un mode d'apprentissage initial ou cyclique dans lequel les canaux de fréquence, plus particulièrement les électrodes sélectionnées (32) et des canaux de fréquence correspondants, ainsi que des critères de signaux d'activité se rapportant de manière sélective à un mouvement donné sont déterminés dans les canaux de fréquence en particulier des électrodes sélectionnées (32).

15. Dispositif BCI (10) selon la revendication 2 lequel présente une unité d'amplification/stimulation (22) pourvue d'un système de fixation situé sur le corps du patient (12) et d'une connexion avec un système informatique (24) équipé de l'unité d'évaluation et relié en particulier au système d'affichage (26) ou à l'orthèse (28), la sonde (18) étant réalisée sous forme d'implant destiné à être disposé de manière durable sous la calotte crânienne et présentant une interface sans fil (20) assurant la connexion avec une interface sans fil de l'unité d'amplification/stimulation (22).
